(19) Europäisches Patentamt
European Patent Office
Office européen des brevets

(11) Numéro de publication: **0 558 933 A1**

(12) **DEMANDE DE BREVET EUROPEEN**

(21) Numéro de dépôt: **93101499.7**

(22) Date de dépôt: **01.02.93**

(51) Int. Cl.[5]: **C07C 49/643**, C07C 49/453, A61K 7/46, C11B 9/00, C07C 45/72, C07C 45/62

(30) Priorité: **04.03.92 CH 680/92**

(43) Date de publication de la demande: **08.09.93 Bulletin 93/36**

(84) Etats contractants désignés: **CH DE FR GB LI NL**

(71) Demandeur: **FIRMENICH SA**
**1, route des Jeunes**
**CH-1211 Genève 8(CH)**

(72) Inventeur: **Giersch, Wolfgang Klaus**
**323, rue de Bernex**
**CH-1233 Bernex(CH)**
Inventeur: **Schulte-Elte, Karl-Heinrich**
**44, chemin de Carabot**
**CH-1213 Onex(CH)**

(74) Mandataire: **Salvadori, Giuseppe, Dr. et al**
**c/o Firmenich S.A. Case Postale 239**
**CH-1211 Genève 8 (CH)**

(54) **Cétones tricycliques, procédé pour leur préparation et leur utilisation en tant qu'ingrédients parfumants.**

(57) Les composés tricycliques spiranniques de formule

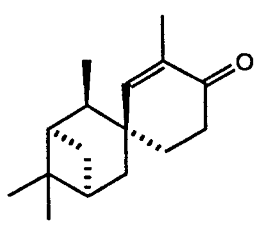

(Ia)

ou

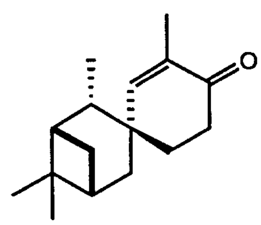

(Ib)

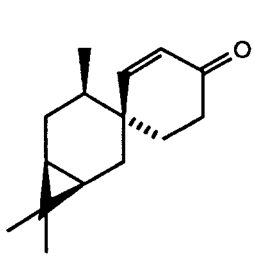

(IIa)

ou

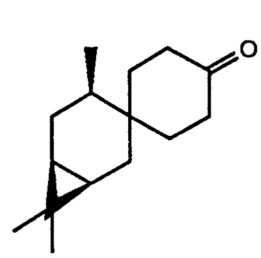

(IIb)

EP 0 558 933 A1

possèdent des propriétés odorantes utiles et trouvent de ce fait une utilisation intéressante en parfumerie, notamment pour conférer, renforcer ou modifier des notes racineuses qui évoquent tout particulièrement la note du vetyver.
Les composés de l'invention sont préparés à partir de (-) ou (+)-3-formyl-pynane, ou de (-)-4-formyl-carane, par une réaction d'addition avec l'éthyl-vinyl cétone ou respectivement la méthyl-vinyl cétone suivie, le cas échéant, d'une réduction par hydrogénation catalytique.

La présente invention a trait au domaine de la parfumerie. Elle concerne plus particulièrement les cétones tricycliques de formule

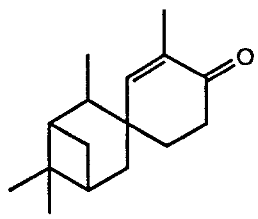

(I)

et de formule

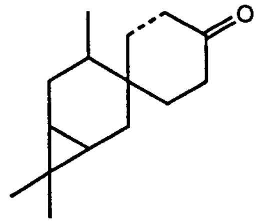

(II)

laquelle formule (II) peut posséder une liaison simple ou double dans la position indiquée par les pointillés. La demande de brevet européen 382 934, publiée le 22.08.90, décrit des cétones tricycliques de formule

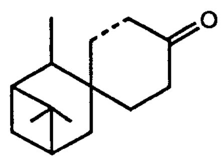

(a)

ainsi que leur emploi en tant qu'agents parfumants. A titre de composés définis à l'aide de la formule (a) la demande précitée revendique en particulier la (+)-(1S,2S,3S)-2,6,6-triméthyl-bicyclo[3.1.1]heptane-3-spiro-1'-(2'-cyclohexén-4'-one) et la (+)-(1R,2R)-2,6,6-triméthyl-bicyclo-[3.1.1]heptane-3-spiro-1'-(4'-cyclohexanone) comme étant des ingrédients de choix.

Nous avons maintenant découvert de manière fortuite que d'autres composés apparentés aux cétones tricycliques de l'art antérieur possédaient des caractères olfactifs intéressants et, qui plus est, différents de ceux montrés pour lesdits composés connus. En effet tout en développant des notes boisées, les nouveaux composés de l'invention servent à conférer, renforcer ou modifier des notes racineuses qui évoquent tout particulièrement la note du vetyver ou encore des notes cédrées, douces presque vanillées avec des nuances balsamiques. De tels caractères sont nettement distincts de ceux des composés de formule (a) et permettent ainsi d'enrichir la palette du parfumeur notamment pour la reconstitution de la note du vetyver Bourbon, une essence ayant presque disparu de la parfumerie moderne à cause de sa rareté.

La présente invention a pour objet tout particulièrement les composés de formule

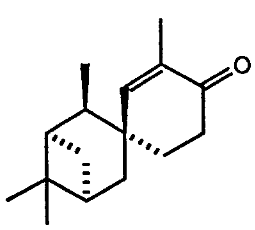

ou

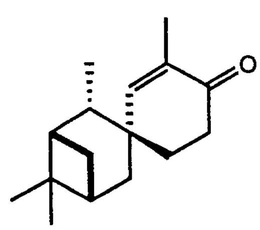

(Ia) (Ib)

définis comme étant respectivement la (-)-(1R,2R,3S)-2,3',6,6-tétraméthyl-bicyclo[3.1.1]heptane-3-spiro-1'-(2'-cyclohexén-4'-one) et la (+)-(1S,2S,3R)-2,3',6,6-tétraméthyl-bicyclo[3.1.1]heptane-3-spiro-1'-(2'-cyclohexén-4'-one), et de formule

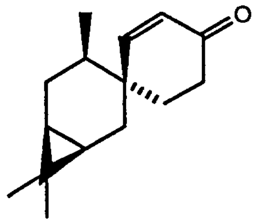

ou

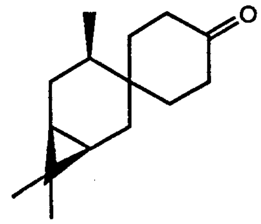

(IIa) (IIb)

définis comme étant respectivement la (+)-(1R,3S,4R)-4,7,7-triméthyl-bicyclo[4.1.0]heptane-3-spiro-1'-(2'-cyclohexén-4'-one) et la (-)-(1R,4R)-4,7,7-triméthyl-bicyclo[4.1.0]heptane-3-spiro-1'-(cyclohexan-4'-one).

Le premier de ces composés, à savoir le composé (Ia), possède une note boisée et douce, balsamique, presque vanillée, tandis que le composé (IIa) est caractérisé par une note boisée avec un caractère légèrement rhubarbe et une nuance rappelant les racines de vetyver, une note qui se retrouve également dans le composé (IIb). Le composé (Ib) pour sa part possède une jolie note boisée, cédrée, légèrement moins puissante que celle de son énantiomère (Ia).

Au vu de leurs propriétés, les composés de l'invention peuvent être employés pour la manufacture de parfums et bases parfumantes ou pour le parfumage de produits de consommation variés, tels les savons, les cosmétiques, les shampoings, les détergents, les produits d'entretien ou les désodorisants corporels ou d'air ambiant. Les composés de l'invention trouvent également une utilisation dans le parfumage de matériaux polymériques, tels ceux qui sont communément utilisés à titre de support pour des dispositifs désodorisants ou assainissants d'air ambiant ou d'enceintes fermées.

Les proportions dans lesquelles les composés de l'invention peuvent développer les caractères odorants désirés peuvent varier dans une gamme de valeurs très étendue. L'homme de l'art sait par expérience que de telles valeurs dépendent de l'effet particulier recherché ainsi, bien entendu, que de la nature des produits que l'on désire parfumer. On sait également que ces proportions sont fonction de la nature des autres constituants dans une composition donnée lorsque les composés de formule (I) et (II) sont utilisés en tant qu'ingrédients dans une base parfumante ou dans un concentré en mélange avec d'autres ingrédients, des dissolvants ou des adjuvants usuels. Par autre ingrédient il faut entendre tout composé d'origine naturelle ou synthétique dont l'emploi en parfumerie est courant ou tout au moins documenté. Il peut s'agir de composés appartenant à des classes chimiques variées telles les cétones, les aldéhydes, les alcools ou les esters en particulier. La seule limitation dans le choix que le parfumeur doit opérer parmi de tels produits est constituée par l'équilibre olfactif de la composition résultante.

Parmi les coingrédients usuels, il convient de citer ceux mentionnés dans les ouvrages techniques tels par exemple S. Arctander, Perfume and Flavor Chemicals, Mont-Clair, N.J. (1969).

Compte tenu de ce qui précède, les composés de l'invention peuvent, par exemple, être employés dans des proportions de l'ordre de 5 à 10%, voire 20% en poids par rapport au poids total de la composition dans laquelle ils sont incorporés. Des valeurs inférieures sont utilisées lors du parfumage de produits tels les savons ou les détergents pour lesquels des effets intéressants peuvent déjà être obtenus par l'adjonction des composés de l'invention dans des proportions de l'ordre de 0,1 à 0,5%, voire 1% en poids.

La présente invention a donc pour objet l'utilisation d'un des composés définis par la formule (I) ou (II) et plus particulièrement par les formules (Ia), (Ib), (IIa) et (IIb), à titre d'ingrédient parfumant actif.

Elle concerne également un produit résultant de ladite utilisation tel une base parfumée, un parfum, un shampoing, un cosmétique, un savon, un détergent, un adoucisseur textile, un désodorisant corporel ou d'air ambiant ou un produit d'entretien.

Les composés objet de la présente invention, sont des composés nouveaux qui peuvent être obtenus à partir de 3-formyl-pinane et 4-formyl-carane selon les schémas réactionnels que voici.

Schéma I

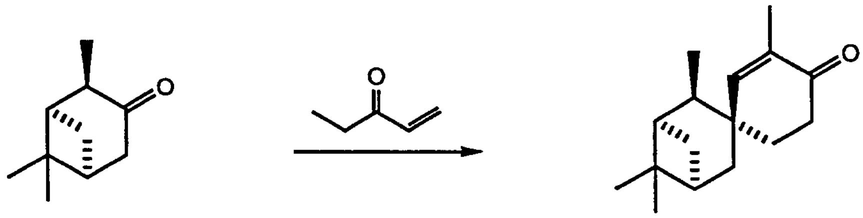

(-)-3-formyl-pinane (Ia)

L'utilisation de (+)-3-formyl-pinane en tant que produit de départ dans ce schéma permet d'obtenir le composé (Ib).

Schéma II

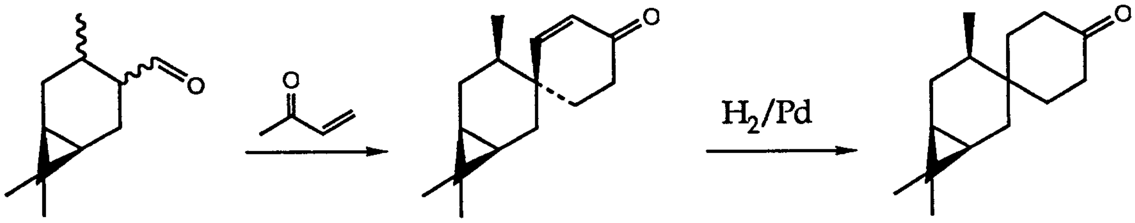


(-)-4-formyl-carane (IIa) (IIb)

La présente invention a également pour objet un procédé pour la préparation desdits composés (Ia, b) et (IIa, b), lequel procédé est caractérisé en ce que :

(a) on additionne à du (-)-3-formyl-pinane, respectivement (+)-3-formyl-pinane, de l'éthyl-vinyl cétone pour fournir la (-)-(1R,2R,3S)-2,3',6,6-tétraméthyl-bicyclo[3.1.1]heptane-3-spiro-1'-(2'-cyclohexén-4'-one), respectivement la (+)-(1S,2S,3R)-2,3',6,6-tétraméthyl-bicyclo [3.1.1]heptane-3-spiro-1'-(2'-cyclohexén-4'-one), ou

(a') on additionne à du (-)-4-formyl-carane de la méthyl-vinyl cétone pour fournir de la (+)-(1R,3S,4R)-4,7,7-triméthyl-bicyclo[4.1.0]heptane-3-spiro-1'-(2'-cyclohexén-4'-one), et

(b') on soumet le composé ainsi obtenu à une hydrogénation catalytique pour donner la (-)-(1R,4R)-4,7,7-triméthyl-bicyclo[4.1.0]heptane-3-spiro-1'-(cyclohexan-4'-one).

L'addition de la méthyl-vinyl cétone ou de l'éthyl-vinyl cétone sur le 4-formyl-carane, respectivement 3-formyl-pinane, s'effectue dans des conditions de réaction courantes. De préférence les réactifs en solution alcoolique basique sont chauffés, après addition, à la température d'ébullition ou à une température proche de celle-ci. Le produit d'addition résultant peut être séparé du mélange réactionnel par simple distillation.

L'étape (b') du procédé, qui consiste en la réduction par hydrogénation catalytique, est effectuée en soumettant la cétone résultant de l'étape (a') à l'action de l'hydrogène dans un réacteur à pression atmosphérique en présence d'un catalyseur métallique usuel, par exemple le palladium, de préférence sur charbon actif, ou le $PtO_2$.

La méthode particulière suivie sera décrite dans le détail dans les exemples spécifiques donnés ci-après. Dans lesdits exemples, les températures sont indiquées en degrés centigrades et les abréviations ont le sens usuel dans l'art.

Exemple 1

Préparation de (+)-(1R,3S,4R)-4,7,7,-triméthyl-bicyclo[4.1.0]heptane-3-spiro-1'-(2'-cyclohexén-4'-one)

50,0 G de 4-formyl-carane [origine : DRT, Dax, France; $[\alpha]^{20}_D$ = -65,8°], ont été mélangés avec 45 g de méthyl-vinyl cétone dans 150 ml d'éthanol en présence de 2 g de KOH dans un réacteur comportant un réfrigérant et un agitateur. Le mélange résultant a été ensuite chauffé à reflux pendant 5 h. Après refroidissement, on a concentré le tout à pression réduite et on a repris le résidu avec de l'éther de pétrole 30/50, puis on a lavé 3 fois avec de l'eau. Après élimination du solvant, on a distillé sous vide et obtenu 13 g (rend. 20%) d'une fraction ayant P. éb. env. 100°/13,3 Pa.
$[\alpha]^{20}_D$ = +2,5°
RMN($^1$H,360MHz) : 0,79(3H, d, J=7); 0,92 et 0,95(6H, 2s); 5,8 et 6,53(2H, 2d, J=10) δppm.
SM : $M^+$ = 218(12); m/z : 203(4), 175(12), 122(100), 107(46), 96(69), 81(97), 67(41), 55(41), 41(47).

Exemple 2

Préparation de (+)-(1S,2S,3R)-2,3',6,6-tétraméthyl-bicyclo[3.1.1]heptane-3-spiro-1'-(2'-cyclohexén-4'-one) et de (-)-(1R,2R,3S)-2,3',6,6-tétraméthyl-bicyclo[3.1.1]heptane-3-spiro-1'-(2'-cyclohexén-4'-one)

27 G (163 mmoles) de (+)-3-formyl-pinane [origine : Firmenich SA, Genève, Suisse; $[\alpha]^{20}_D$ = +19,5°] ont été mélangés avec 20 g (238 mmoles) d'éthyl-vinyl cétone [origine : Fluka AG, Bucks, Suisse] dans 100 ml d'éthanol et en présence de 2g de KOH. Le mélange obtenu a été porté à la température d'ébullition et maintenu à reflux pendant 2 1/2 h, puis concentré sous vide. Repris ensuite avec de l'éther, extrait avec 2 fractions d'eau et concentré à nouveau avant distillation. On a ainsi obtenu 27 g (rend. 72%) de (+)-(1S,2S,3R)-2,3',6,6-tétraméthyl-bicyclo[3.1.1]heptane-3-spiro-1'-(2'-cyclohexén-4'-one) ayant P. éb. 90°/20 Pa.
$[\alpha]^{20}_D$ = +44° (EtOH, c = 10%)
RMN($^1$H, 360MHz) : 1,01(3H, d, J=7); 1,015(3H, s); 1,24(3H, s); 1,79(3H, s); 6,87(1H, s) δppm.
RMN($^{13}$C, 360MHz) : 199,8(s); 155,3(d); 132,7(s); 48,6(d); 45,9(t); 44,1(d); 41,8(d); 40,4(t); 38,5(s); 35,1(s); 34,5(t); 29,5(t); 27,8(q); 23,2(q); 19,9(q); 16,2(q).
SM : $M^+$ = 232(0,1); m/z : 189(1), 177(5), 164(16), 150(17), 123(47), 110(49), 95(100), 83(33), 55(33), 41-(28).
Suivant la même méthode, à partir de (-)-3-formyl-pinane [25 g; $[\alpha]^{20}_D$ = -11,6°; origine : Firmenich SA, Genève, Suisse] on a obtenu 21,3 g (rend. 71%) de (-)-(1R,2R,3S)-2,3',6,6-tétraméthyl-bicyclo[3.1.1]-heptane-3-spiro-1'-(2'-cydohexén-4'-one) ayant P. éb. 100°/30 Pa et $[\alpha]^{20}_D$ = -43° (EtOH, c = 10%). Les autres données analytiques de ce composé étaient identiques à celles indiquées ci-dessus pour son énantiomère.

Exemple 3

Préparation de (-)-(1R,4R)-4,7,7-triméthyl-bicyclo[4.1.0]heptane-3-spiro-1'-(cyclohexan-4'-one)

5,0 G du produit obtenu à l'exemple 1 dans 50 ml d'acétate d'éthyle ont été soumis à une hydrogénation catalytique à pression atmosphérique en présence de 0,2 g de palladium à 10% sur charbon actif. La réaction a été poursuivie jusqu'à cessation de l'absorption d'hydrogène. Les traitements habituels de filtration, concentration et distillation ont fourni la cétone désirée à éb. 150° (temp. du bain)/26,6 Pa; 4,5g (rend. 89,3%). F. 52-3° après recristallisation dans l'éther de pétrole 80/100.
$[\alpha]^{20}_D$ = -29,5° (EtOH, c = 10%)
RMN($^1$H, 360MHz) : 0,845(3H, d, J=7); 0,98(3H, s); 1,03(3H, s) δppm.
SM : $M^+$ = 220(12); 205(2), 177(10), 163(22), 136(27), 107(41), 96(71), 81(100), 67(58), 55(67), 41(51).

Exemple 4

On a préparé une composition de base pour une ligne de parfums féminins en mélangeant les ingrédients suivants (parties en poids) :

| Ingrédients | Parties en poids |
|---|---|
| Acétate de carbinyl à 10% * | 200 |
| Acétate de géranyl | 50 |
| Acétate de linalyl | 1200 |
| Muscone 1) | 100 |
| 4-(4-Hydroxy-phényl)-2-butanone à 10% * | 30 |
| Essence de Bergamote Abergapt | 600 |
| Citral | 120 |
| Essence de citron SFUMA | 1500 |
| Galaxolide ® 50 2) | 250 |
| Exaltex ® 1) | 100 |
| Essence de girofle, griffes | 300 |
| Essence de lavande | 600 |
| Linalol | 200 |
| Lyral ® 2) | 1000 |
| Essence de marjolaine douce | 350 |
| Mousse de chêne absolue à 50% * | 150 |
| Muscade | 500 |
| Salicylate de benzyle | 50 |
| Sandela ® 3) | 600 |
| Ylang | 300 |
| Essence de galbanum à 10% * | 300 |
| Kephalis 3) | 500 |
| Total | 9000 |

* dans le phtalate de diéthyle
1) origine : Firmenich SA, Genève, Suisse
2) origine : International Flavors & Fragrances Inc., USA
3) origine : Givaudan-Roure, Vernier, Suisse

Lorsqu'on ajoute à 90 parties en poids de la composition de base définie ci-dessus, 10 parties en poids de (+)-(1R,3S,4R)-4,7,7-triméthyl-bicyclo[4.1.0]heptane-3-spiro-1'-(2'-cyclohexén-4'-one), on obtient une nouvelle composition dont le caractère boisé se trouve renforcé par une note racineuse évoquant le vetyver tout en possédant un aspect balsamique et floral.

Exemple 5

On a préparé une composition de base en mélangeant les ingrédients définis dans l'exemple précédent dans les mêmes proportions.
Lorsqu'on ajoute à 90 parties en poids de la composition de base 10 parties en poids de (-)-(1R,2R,3S)-2,3',6,6-tétraméthyl-bicyclo[3.1.1]heptane-3-spiro-1'-(2'-cyclohexén-4'-one) on obtient une nouvelle composition dont le caractère est plus doux et balsamique avec un aspect vanillé, ce qui lui confère une note globalement plus raffinée.

Exemple 6

On a préparé une composition parfumante de base, destinée à un détergent en poudre, en mélangeant les ingrédients suivants (parties en poids) :

| Ingrédients | Parties en poids |
|---|---|
| Acétate de carbinol BDM | 110 |
| Aldéhyde hexylcinnamique | 3 250 |
| Acétate d'isononyle | 260 |
| Propanoate d'éthyl tricyclo[5.2.1.0$^{2,6}$]déc-3-én-8-yle 1) | 830 |
| Coumarine | 80 |
| Aldéhyde cyclamen | 400 |
| Acétate de 4-tert-butylcyclohexyle 2) | 1 100 |
| Fleuramone ® 3) | 240 |
| Florol ® 4) | 30 |
| Galaxolide ® 50 5) | 260 |
| Iralia ® 6) | 220 |
| Lilial ® 7) | 840 |
| Phenylhexanol | 520 |
| Salicylate de benzyle | 340 |
| Tétrahydrolinalol | 360 |
| Tonalid ® 8) | 900 |
| Verdox ® 9) | 260 |
| Total | 10 000 |

1) origine : Firmenich SA, Genève, Suisse
2) origine : Firmenich SA, Genève, Suisse
3) 2-heptyl-1-cyclopentanone ; origine : International Flavors & Fragrances Inc., USA
4) tétrahydro-2-isobutyl-4-méthyl-4-(2H)-pyranol ; origine : Firmenich SA, Genève, Suisse
5) 1,3,4,6,7,8-hexahydro-4,6,6,7,8,8-hexaméthyl-cyclopenta[g]isochromène ; origine : International Flavors & Fragrances Inc., USA
6) méthyl-α-ionone ; origine : Firmenich SA, Genève, Suisse
7) 3-(4-tert-butyl-1-phényl)-2-méthylpropanal ; origine : Givaudan-Roure, Vernier, Suisse
8) 7-acétyl-1,1,3,4,4,6-hexaméthyltétraline ; origine : PFW, Hollande
9) acétate de 2-tert-butylcyclohexanyle ; origine : International Flavors & Fragrances Inc., USA

Lorsqu'on ajoute à 100 parties en poids de cette composition de base de type floral, poudré, musqué, 10 parties en poids de (+)-(1R,3S,4R)-4,7,7-triméthyl-bicyclo[4.1.0]heptane-3-spiro-1'-(2'-cyclohexén-4'-one), on obtient une composition nouvelle ayant un caractère très boisé, balsamique et floral, avec une sous-note racineuse évoquant le vetyver.

D'autre part, lorsqu'on ajoute à 100 parties en poids de la composition de base 10 parties en poids de (-)-(1R,2R,3S)-2,3',6,6-tétraméthyl-bicyclo[3.1.1]heptane-3-spiro-1'-(2'-cyclohexén-4'-one), on obtient une composition nouvelle boisée, avec un côté plus doux et balsamique, dont la note est globalement plus raffinée.

Des effets olfactifs identiques ont été observés même lorsque l'on a utilisé 5 parties en poids des composés de l'invention, pour 100 parties en poids de composition.

## Revendications

1. Cétone tricyclique de formule

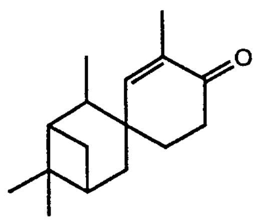

(I)

ou de formule

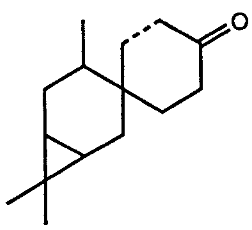

(II)

laquelle formule (II) possède une liaison simple ou double dans la position indiquée par la ligne pointillée.

2. (-)-(1R,2R,3S)-2,3',6,6-Tétraméthyl-bicyclo[3.1.1]heptane-3-spiro-1'-(2'-cyclohexén-4'-one).

3. (+)-(1R,3S,4R)-4,7,7-Triméthyl-bicyclo[4.1.0]heptane-3-spiro-1'-(2'-cyclohexén-4'-one).

4. (-)-(1R,4R)-4,7,7-Triméthyl-bicyclo[4.1.0]heptane-3-spiro-1'-(cyclohexan-4'-one).

5. (+)-(1S,2S,3R)-2,3',6,6-Tétraméthyl-bicyclo[3.1.1]heptane-3-spiro-1'-(2'-cyclohexén-4'-one).

6. Utilisation d'un composé défini dans l'une quelconque des revendications précédentes à titre d'ingrédient parfumant actif.

7. A titre de produit résultant de l'utilisation selon la revendication 6, une base parfumée, un parfum, un shampoing, un cosmétique, un savon, un détergent, un adoucisseur textile, un désodorisant corporel ou d'air ambiant ou un produit d'entretien.

8. Procédé pour la préparation d'un composé selon l'une quelconque des revendications 2 à 5, caractérisé en ce que :
(a) on additionne à du (-)-3-formyl-pinane, respectivement (+)-3-formyl-pinane, de l'éthyl-vinyl cétone pour fournir la (-)-(1R,2R,3S)-2,3',6,6-tétraméthyl-bicyclo[3.1.1]heptane-3-spiro-1'-(2'-cyclohexén-4'-one), respectivement la (+)-(1S,2S,3R)-2,3',6,6-tétraméthyl-bicyclo[3.1.1]heptane-3-spiro-1'-(2'-cyclohexén-4'-one) ou

(a') on additionne à du (-)-4-formyl-carane de la méthyl-vinyl cétone pour fournir de la (+)-(1R,3S,4R)-4,7,7-triméthyl-bicyclo[4.1.0]heptane-3-spiro-1'-(2'-cyclohexén-4'-one), et
(b') on soumet le composé ainsi obtenu à une hydrogénation catalytique pour donner la (-)-(1R,4R)-4,7,7-triméthyl-bicyclo[4.1.0]heptane-3-spiro-1'-(cyclohexan-4'-one).

Office européen des brevets

# RAPPORT DE RECHERCHE EUROPEENNE

Numero de la demande

EP 93 10 1499 · 7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 382 934 (FIRMENICH SA)<br>* revendications *<br>----- | 1,6-8 | C07C49/643<br>C07C49/453<br>A61K7/46<br>C11B9/00<br>C07C45/72<br>C07C45/62 |
| | | | **DOMAINES TECHNIQUES RECHERCHES (Int. Cl.5)** |
| | | | C07C |

Le présent rapport a été établi pour toutes les revendications

| Lieu de la recherche | Date d'achèvement de la recherche | Examinateur |
|---|---|---|
| LA HAYE | 14 JUILLET 1993 | BONNEVALLE E.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant